# EUROPEAN PATENT SPECIFICATION

(11) **EP 3 950 672 B1**
(45) Date of publication and mention of the grant of the patent: **29.07.2026**
(21) Application number: 20777827.5
(22) Date of filing: 21.02.2020
(51) Int. Cl.: C07C 405/00, C07D 307/935

(54) **METHOD FOR PRODUCING PROSTAGLANDIN**
VERFAHREN ZUR HERSTELLUNG VON PROSTAGLANDIN
PROCÉDÉ DE PRODUCTION DE PROSTAGLANDINE

(30) Priority: 27.03.2019 JP 2019060295
(43) Date of publication of application: 09.02.2022
(73) Proprietor: Kyowa Pharma Chemical Co., Ltd., Takaoka-shi, Toyama 933-8511 (JP)
(72) Inventor: ASANO Tatsuhiro, Takaoka-shi, Toyama 933-8511 (JP); MAENISHI Ryota, Takaoka-shi, Toyama 933-8511 (JP); TAKENAKA Junpei, Takaoka-shi, Toyama 933-8511 (JP); ATAGO Takayuki, Machida-shi, Tokyo 194-8533 (JP); NARUMI Chihiro, Suntou-gun, Shizuoka 411-8731 (JP)
(74) Representative: Vossius & Partner Patentanwälte Rechtsanwälte mbB
(86) International application number: PCT/JP2020/007196
(87) International publication number: WO 2020/195437

(56) References cited:
- EP-A1- 1 886 992
- WO-A1-2012/011128
- WO-A1-2012/011128
- WO-A1-2013/093528
- WO-A1-2015/133405
- WO-A2-02/096898
- JP-A- 2005 503 354
- JP-A- 2008 037 782
- JP-A- 2015 506 343
- US-A1- 2016 362 369
- MATTHEW D. NORRIS ET AL: "Biomimetic Total Synthesis of Gracilioethers B and C", ORGANIC LETTERS, vol. 17, no. 3, 26 January 2015 (2015-01-26), US, pages 668 - 671, XP055742727, ISSN: 1523-7060, DOI: 10.1021/ol503695j
- KAZUHIKO MATSUMURA ET AL: "Chiral Ruthenabicyclic Complexes: Precatalysts for Rapid, Enantioselective, and Wide-Scope Hydrogenation of Ketones", JOURNAL OF THE AMERICAN CHEMICAL SOCIETY, vol. 133, no. 28, 20 July 2011 (2011-07-20), pages 10696 - 10699, XP055131776, ISSN: 0002-7863, DOI: 10.1021/ja202296w
- NORRIS, M. D. ET AL.: "Biomimetic Total Synthesis of Gracilioethers B and C", ORG. LETT., vol. 17, 2015, pages 668 - 671, XP055742727
- MATSUMURA, K. ET AL.: "Chiral Ruthenabicyclic Complexes: Precatalysts for Rapid, Enantioselective, and Wide-Scope Hydrogenation of Ketones", J. AM. CHEM. SOC., vol. 133, 2011, pages 10696 - 10699, XP055131776, DOI: 10.1021/ja202296w
- COMAN, S. ET AL.: "Low metal loading Ru-MCM-41 stereocontrolled hydrogenation of prostaglandin intermediates", CHEM. COMMUN., pages 2175 - 2176, XP055742729

## Description

### Technical Field

The present invention relates to a method for producing prostaglandins.

### Background Art

Prostaglandin (PG) is a general term for a group of endogenous physiologically active substances synthesized by the metabolism of arachidonic acid in vivo with cyclooxygenase. There are many types of prostaglandins, and, for example, prostaglandin H2, prostaglandin D2, prostaglandin E1, prostaglandin E2, prostaglandin F2α, and prostaglandin I2 are known. Prostaglandins are involved in a variety of physiological functions via their specific G protein-coupled receptors.

The chemical structure of prostaglandins includes a cyclopentane ring having 4 asymmetric carbon atoms and 2 aliphatic side chains. Therefore, it has been focused upon as a target for synthesis studies or an idea for drug development for a long time, and various prostaglandin derivatives have been developed so far. (3aS,4R,5S,6aR)-(+)-hexahydro-5-hydroxy-4-(hydroxymethyl)-2H-cycl openta[b]furan-2-one used as its common intermediate is also called "Corey lactone." The chemical structures of Corey lactone and typical commercially available prostaglandin derivatives are shown below.

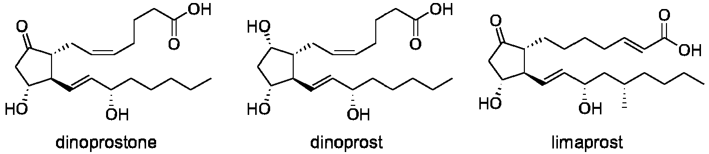

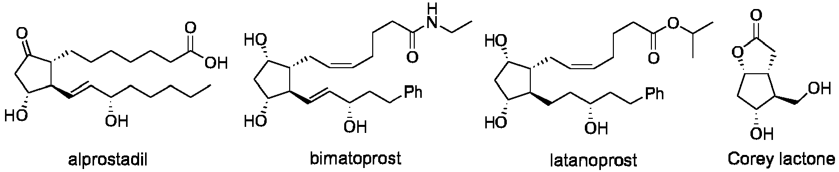

After development of Corey lactones, the main challenge in the synthesis of prostaglandin derivatives is control of the stereochemistry of the substituents present in side chains. In particular, more studies have been conducted in order to introduce hydroxy groups having a desired stereochemistry. In Patent Literature 1, a method in which corresponding carbonyl groups are reduced and diastereomers are then separated through column chromatography and a method in which substituents are additionally introduced at hydroxy groups generated by the reduction, and the optical purity is increased by crystallization or the like have been reported. Patent Literature 2 discloses a method in which side chain units having a desired stereochemistry are bonded to cyclopentane derivatives according to a coupling reaction. In Patent Literature 3, reduction of corresponding carbonyl groups using an optically active reducing agent such as (-)-DIP-chloride^{™} has been studied.
Patent Literature 10 discloses that lithium aluminum hydride and (S)-1,1-bi-naphthol are used for the asymmetric reduction step in the preparation of latanoprost. Patent Literature 11 discloses that catecholborane and R-(+)2-methyl-CBS-oxazaborolidine are used for the asymmetric reduction step in the preparation of travoprost. Further, in Patent Literature 5, a Corey catalyst and a borane-dimethylsulfide complex are used for the asymmetric reduction Step in the preparation of latanoprost.

### Citation List

### Patent Literature

[Patent Literature 1] WO 2012/011128 A1
[Patent Literature 2] US 2007/167641 A1
[Patent Literature 3] CN 105985371 A1
[Patent Literature 4] EP 2837621 A1
[Patent Literature 5] WO 2002/096898 A2
[Patent Literature 6] WO 2007/091697 A2
[Patent Literature 7] US 6248783 B1
[Patent Literature 8] US 3726983 B1
[Patent Literature 9] US 2009/259066 A1
[Patent Literature 10] EP 1 886 992 A1
[Patent Literature 11] WO 2013/093528 A1

### Non Patent Literature

[Non-Patent Literature 1] E. J. Corey et al., J. Am. Chem. Soc., 1969, 91, 5675.

### Summary of Invention

### Technical Problem

However, in the above methods, it is necessary to perform purification through column chromatography, increase the number of steps according to derivatization, and use a stoichiometric amount of an optically active reagent, and there are concerns about the economic burden and environmental burden. Therefore, an object of the present invention is to provide a method for efficiently producing prostaglandins using an optically active catalyst and a synthetic intermediate thereof.

### Solution to Problem

The present invention provides the following [1] to [5].
[1] A method for producing a compound represented by Formula (1a), (1b), or (1c): 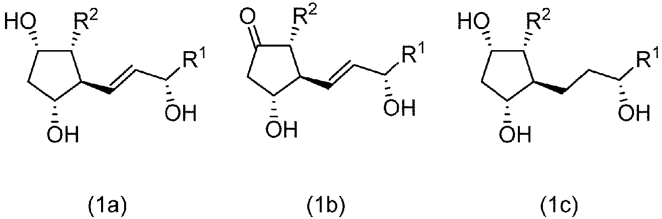
   in the formula, R¹ is a C₁₋₈ alkyl group that is optionally substituted with a phenyl group, R² is a group represented by Formula (2a), (2b), or (2c): 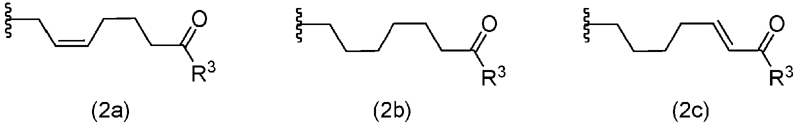
   in the formula, R³ is a hydroxy group, a C₁₋₃ alkoxy group, a mono(C₁₋₃ alkyl)amino group or a di(C₁₋₃ alkyl)amino group;
   the method comprising a step of reducing a compound represented by Formula (3) in the presence of a metal complex represented by Formula (5), an inorganic base, and a solvent under a hydrogen atmosphere to obtain a compound represented by Formula (4): 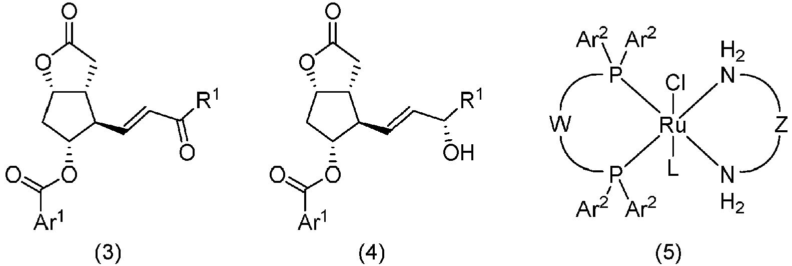
   in the formula, Ar¹ is an aryl group that is optionally substituted,
   each Ar² is independently a phenyl group, a 3,5-dimethylphenyl group, or a 3,5-di(tert-butyl)-4-methoxyphenyl group,
   W is a biphenyl group that is optionally substituted or a binaphthyl group that is optionally substituted,
   Z is an ethylene group that is substituted with two or more groups selected from among a phenyl group, a C₁₋₃ alkoxyphenyl group and a C₁₋₈ alkyl group, and L is a chlorine atom or if Z has a phenyl group or a C₁₋₃ alkoxyphenyl group, L is one of carbon atoms constituting the phenyl group or the C₁₋₃ alkoxyphenyl group.
[2] The method according to [1], wherein the solvent comprises at least one solvent selected from among ethers, alcohols, acetonitrile and water.
[3] The method according to [1] or [2], wherein Ar¹ is a phenyl group, and Ar² is a 3,5-dimethylphenyl group.
[4] The method according to any of [1] to [3], wherein R¹ is a n-pentyl group, a 2-methylhexyl group, or a 2-phenylethyl group.
[5] The method according to any of [1] to [4], further comprising a step of reacting a compound represented by Formula (4) with a compound represented by Formula (6) to obtain a compound represented by Formula (7): 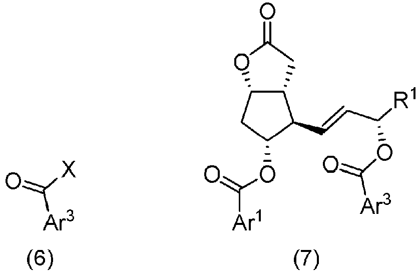 in the formula, R¹ is a C₁₋₈ alkyl group that is optionally substituted with a phenyl group, Ar¹ and Ar³ are each independently an aryl group, and X is a leaving group.

### Advantageous Effects of Invention

According to the present invention, when catalytic hydrogen reduction using an optically active catalyst is performed, it is possible to introduce hydroxy groups on the side chain with high stereoselectivity and it is possible to efficiently produce prostaglandin derivatives.

### Description of Embodiments

An embodiment of the present invention will be described in detail. In this specification, for convenience, a "compound represented by Formula (1)" and the like are referred to as a "compound (1)" and the like.

An embodiment of the present invention is a method for producing a compound (1a), (1b), or (1c).

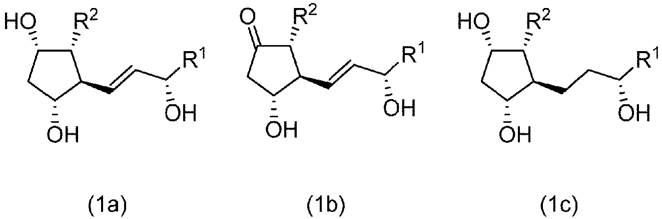

In Formula (1a), (1b), or (1c), R¹ is a C₁₋₈ alkyl group that is unsubstituted or substituted with a phenyl group. The C₁₋₈ alkyl group is a linear or branched alkyl group having 1 to 8 carbon atoms and may be an optically active branched alkyl group. Examples of C₁₋₈ alkyl groups include a methyl group, an ethyl group, a 1-propyl group, a 2-propyl group (also referred to as an isopropyl group), a 1-butyl group, a 2-methyl-1-propyl group (also referred to as an isobutyl group), a 1-methyl-2-propyl group, a tert-butyl group, a 1-pentyl group, a 2-methyl-1-butyl group, a 3-pentyl group, a 3-methyl-1-butyl group (also referred to as an isopentyl group), a 2,2-dimethyl-1-propyl group (also referred to as a neopentyl group), a 1-hexyl group, a 1-methyl-2-pentyl group, a 2-methyl-2-pentyl group, a 3-methyl-2-pentyl group, a 1-heptyl group, a 2-heptyl group, a 2-methyl-1-hexyl group, a 2-ethyl-1-pentyl group, a 3-methyl-1-hexyl group, a 3-ethyl-1-pentyl group, 3-heptyl group, a 4-heptyl group, a 1-octyl group, a 2-octyl group, a 2-methyl-1-heptyl group, a 3-ethyl-1-pentyl group, and a 3-propyl-1-butyl group. In addition, the C₁₋₈ alkyl group substituted with a phenyl group is a group in which some of hydrogen atoms constituting the above linear or branched alkyl group having 1 to 8 carbon atoms are replaced with a phenyl group. Specific examples of the C₁₋₈ alkyl group substituted with a phenyl group include a phenylmethyl group (also referred to as a benzyl group), a 1-phenylethyl group, a 2-phenylethyl group, a 1-phenylpropyl group, and a 1-methyl-1-phenyl ethyl group.

In Formula (1a), (1b), or (1c), R² is a group represented by Formula (2a), (2b), or (2c), and R³ is a hydroxy group, a C₁₋₃ alkoxy group, a mono(C₁₋₃ alkyl)amino group, or a di(C₁₋₃ alkyl)amino group.

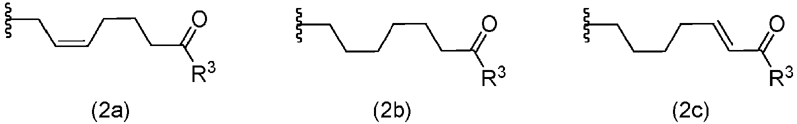

The C₁₋₃ alkoxy group is a group composed of an oxygen atom to which a linear or branched alkyl group having 1 to 3 carbon atoms is bonded. Examples of C₁₋₃ alkoxy groups include a methoxy group, an ethoxy group, a 1-propyloxy group, and a 2-propyloxy group (also referred to as an isopropyloxy group).

The mono(C₁₋₃ alkyl)amino group is a group composed of amino groups to which one linear or branched alkyl group having 1 to 3 carbon atoms is bonded. Examples of mono(C₁₋₃ alkyl)amino groups include a monomethylamino group, a monoethylamino group, a mono(1-propyl)amino group, and a mono(2-propyl)amino group.

The di(C₁₋₃ alkyl)amino group is a group composed of amino groups to which two linear or branched alkyl groups having 1 to 3 carbon atoms are bonded. Examples of di(C₁₋₃ alkyl)amino groups include a N,N-dimethylamino group, a N-ethyl-N-methylamino group, a N,N-diethylamino group, a N-ethyl-N-(1-propyl)amino group, a N,N-di(1-propyl)amino group, and a N,N-di(2-propyl)amino group.

### (Step 1)

The method according to the present embodiment comprises a step of reducing a compound (3) under a hydrogen atmosphere in the presence of a metal complex (5), an inorganic base, and a solvent to obtain a compound (4). In Formula (3) or (4), R¹ has the same definition as in Formula (1), and Ar¹ is an aryl group that is optionally substituted.

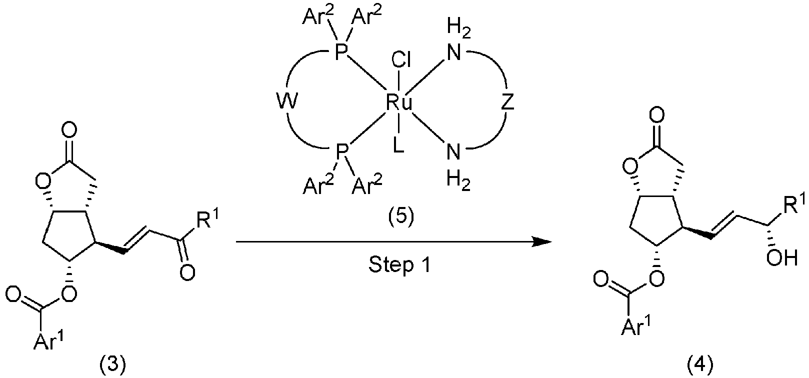

The aryl group is an aromatic hydrocarbon group, and may be, for example, an aromatic hydrocarbon group having 6 to 10 carbon atoms. Examples of aryl groups include a phenyl group and a naphthyl group. The aryl group is optionally substituted with a halogen atom (for example, a fluorine atom, a chlorine atom, a bromine atom, and an iodine atom), a C₁₋₃ alkyl group, a C₁₋₃ alkoxy group, or a cyano group.

In Formula (5), each Ar² is independently a phenyl group, a 3,5-dimethylphenyl group, or a 3,5-di(tert-butyl)-4-methoxyphenyl group. A group in which four Ar²'s are the same group is preferable.

In Formula (5), W is a biphenyl group that is optionally substituted or a binaphthyl group that is optionally substituted. In addition, (Ar²)₂P-W-P(Ar²)₂ is a bidentate ligand that can be coordinated to a ruthenium atom, and W is preferably a bis(methylenedioxyphenyl)group or a binaphthyl group which is a single atropisomer. Examples of (Ar²)₂P-W-P(Ar²)₂ include 2,2'-bis(diphenylphosphino)-1,1'-binaphthyl (BINAP), 2,2'-bis[di(p-tolyl)phosphino]-1,1'-binaphthyl (tolBINAP), 2,2'-bis[di(m-tolyl)phosphino]-1,1'-binaphthyl, 2,2'-bis[di(3,5-xylyl)phosphino]-1,1'-binaphthyl (xylBINAP), 2,2'-bis[di(p-t-butylphenyl)phosphino]-1,1'-binaphthyl, 2,2'-bis[di(p-methoxyphenyl)phosphino]-1,1'-binaphthyl, 2,2'-bis[di(3,5-di-t-butyl-4-methoxyphenyl)phosphino]-1,1'-binaphthyl, 2,2'-bis[di(cyclopentyl)phosphino]-1,1'-binaphthyl, 2,2'-bis[di(cyclohexyl)phosphino]-1,1'-binaphthyl,2,2'-bis(diphenylpho sphino)-5,5',6,6',7,7',8,8'-octahydro-1,1'-binaphthyl, 2,2'-bis(di-p-tolylphosphino)-5,5',6,6',7,7',8,8'-octahydro-1,1'-binapht hyl, 2,2'-bis(di-m-tolylphosphino)-5,5',6,6',7,7',8,8'-octahydro-1,1'-binapht hyl, 2,2'-bis(di-3,5-xylylphosphino)-5,5',6,6',7,7',8,8'-octahydro-1,1'-binap hthyl (xyl-H8-BINAP), 2,2'-bis(di-p-t-butylphenylphosphino)-5,5',6,6',7,7',8,8'-octahydro-1,1' -binaphthyl, 2,2'-bis(di-p-methoxyphenylphosphino)-5,5',6,6',7,7',8,8'-octahydro-1, 1'-binaphthyl, 2,2'-bis(di-p-chlorophenylphosphino)-5,5',6,6',7,7',8,8'-octahydro-1,1' -binaphthyl, 2,2'-bis(dicyclopentylphosphino)-5,5',6,6',7,7',8,8'-octahydro-1,1'-bin aphthyl, 2,2'-bis(dicyclohexylphosphino)-5,5',6,6',7,7',8,8'-octahydro-1,1'-bina phthyl, ((4,4'-bi-1,3-benzodioxol)-5,5'-diyl)bis(diphenylphosphine) (segphos), (4,4'-bi-1,3-benzodioxol)-5,5'-diyl)bis(di(3,5-xylyl)phosphine) (dm-segphos), ((4,4'-bi-1,3-benzodioxol)-5,5'-diyl)bis(di(3,5-di-t-butyl-4-methoxyphe nyl)phosphine), ((4,4'-bi-1,3-benzodioxol)-5,5'-diyl)bis(di(4-methoxyphenyl)phosphine ), ((4,4'-bi-1,3-benzodioxol)-5,5'-diyl)bis(dicyclohexylphosphine), ((4,4'-bi-1,3-benzodioxol)-5,5'-diyl)bis(bis(3,5-di-t-butylphenyl)phosph ine), 2,2'-bis(di-3,5-xylylphosphino)-6,6'-dimethoxy-1,1'-biphenyl (xylyl-MeO-biphep), 2,2'-bis(diphenylphosphino)-6,6'-dimethyl-1,1-biphenyl, 2,2'-bis(di-p-tolylphosphino)-6,6'-dimethyl-1,1'-biphenyl, 2,2'-bis(di-o-tolylphosphino)-6,6'-dimethyl-1,1'-biphenyl, 2,2'-bis(di-m-fluorophenylphosphino)-6,6'-dimethyl-1,1'-biphenyl, 2,2'-bis(diphenylphosphino)-6,6'-dimethoxy-1,1'-biphenyl, 2,2'-bis(di-p-tolylphosphino)-6,6'-dimethoxy-1,1'-biphenyl, 2,2',6,6'-tetramethoxy-4,4'-bis(di-3,5-xylyl phosphino)-3,3'-bipyridine (xylyl-p-phos), 2,2',6,6'-tetramethoxy-4,4'-bis(diphenylphosphino)-3,3'-bipyridine, 2,2',6,6'-tetramethoxy-4,4'-bis(di-p-tolylphosphino)-3,3'-bipyridine, 2,2',6,6'-tetramethoxy-4,4'-bis(di-o-tolylphosphino)-3,3'-bipyridine, 4,12-bis(di-3,5-xylylphosphino)-[2.2]-paracyclophane, 4,12-bis(diphenylphosphino)-[2.2]-paracyclophane, 4,12-bis(di-p-tolylphosphino)-[2.2]-paracyclophane, 4,12-bis(di-o-tolylphosphino)-[2.2]-paracyclophane, 1,1'-bis(2,4-diethylphosphotano)ferrocene, 1,13-bis(diphenylphosphino)-7,8-dihydro-6H-dibenzo [f,h] [1,5]dioxonin, 1,13-bis(bis(3,5-dimethylphenyl)phosphino)-7,8-dihydro-6H-dibenzo[f, h][1,5]dioxonin (xylyl-C3-tunephos), and 6,6'-bis(bis(3,5-dimethylphenyl)phosphino)-2,2',3,3'-tetrahydro-5,5'-bi -1,4-benzodioxine (xylyl-synphos).

In Formula (5), Z may be an ethylene group substituted with two or more groups selected from among a phenyl group, a C₁₋₃ alkoxyphenyl group and a C₁₋₈ alkyl group, and in this case, L is a chlorine atom. H₂N-Z-NH₂ is a bidentate ligand that can be coordinated to a ruthenium atom. Examples of H₂N-Z-NH₂ include (S,R)-1,2-diphenylethylenediamine, (R,S)-1,2-diphenylethylenediamine, 1,2-bis(4-methoxyphenyl)ethylenediamine, 1-methyl-2,2-diphenylethylenediamine, 1-isobutyl-2,2-diphenylethylenediamine, 1-isopropyl-2,2-diphenylethylenediamine (DPIPEN), 1-methyl-2,2-bis(4-methoxyphenyl)ethylenediamine (DAMEN), 1-isobutyl-2,2-bis(4-methoxyphenyl)ethylenediamine, 1-isopropyl-2,2-bis(4-methoxyphenyl)ethylenediamine (DAIPEN), 1-phenyl-2,2-bis(4-methoxyphenyl)ethylenediamine, 1,1-bis(4-methoxyphenyl)ethylenediamine (DAEN), and 1-isopropyl-2,2-bis(3-methoxyphenyl)ethylenediamine (3-DAIPEN). A preferable H₂N-Z-NH₂ is optically active 1,2-diphenylethylenediamine or 1-isopropyl-2,2-di(4-methoxyphenyl)ethylenediamine.

In addition, when Z has a phenyl group or a C₁₋₃ alkoxyphenyl group, one of carbon atoms constituting the phenyl group or C₁₋₃ alkoxyphenyl group may be directly bonded to a ruthenium atom so that a ruthenabicyclo[2.2.1]heptane structure is formed.

In the step 1, the compound (3) is dissolved in a solvent in a predetermined container, an inorganic base is added thereto and a gas phase in the container is replaced with an inert gas. Next, after the metal complex (5) is added thereto, a gas phase is replaced with a hydrogen gas, and the mixture is stirred under a hydrogen atmosphere for a predetermined time. The mixture is preferably vigorously stirred so that hydrogen gas in the gas phase is easily mixed into the reaction solution.

The solvent may be any solvent in which the compound (3) can be dissolved and which does not inhibit catalytic hydrogen reduction. Examples of solvents include aromatic hydrocarbons such as benzene, toluene, and xylene, aliphatic hydrocarbons such as hexane and heptane, halogenated hydrocarbons such as dichloromethane, 1,2-dichloroethane, chlorobenzene, and dichlorobenzene, ethers such as diethyl ether, diisopropyl ether, tetrahydrofuran, methyl tert-butyl ether, cyclopentyl methyl ether, and 2-methyl-tetrahydrofuran, alcohols such as methanol, ethanol, isopropyl alcohol, n-butyl alcohol, 2-butyl alcohol, tert-butyl alcohol, ethylene glycol, propylene glycol, 1,2-propanediol, and glycerin, acetonitrile, N,N-dimethylformamide (DMF) ,and N,N-dimethylacetamide. These solvents may be used alone or may be used by being selectively mixed. Water may be incorporated as long as the compound (3) can still dissolve. A preferable solvent is at least one selected from among ethers, alcohols, acetonitrile and water.

The amount of the solvent used is preferably an amount at which the molar concentration of the compound (3) is 0.05 to 1.5 mol/L and more preferably an amount at which the molar concentration of the compound (3) is 0.1 to 0.9 mol/L.

The inorganic base may be a basic inorganic salt. Examples of inorganic bases include potassium carbonate, sodium carbonate, cesium carbonate, potassium bicarbonate, sodium bicarbonate, potassium hydroxide, sodium hydroxide, tripotassium phosphate, trisodium phosphate, dipotassium hydrogen phosphate, disodium hydrogen phosphate, potassium dihydrogen phosphate, and sodium dihydrogen phosphate.

The amount of the inorganic base used may be 0.1 to 50 equivalents and is more preferably 1 to 20 equivalents with respect to the number of moles of the metal complex (5).

The amount of the metal complex (5) used varies depending on the amount of the compound (3), reaction conditions and the type of the metal complex (5), and is generally 0.1 to 25 mol%, and more preferably 1 to 5 mol% with respect to the number of moles of the compound (3).

The reaction temperature varies depending on the type of the solvent used, and the reaction can be performed at -50 to 50°C. A preferable reaction temperature is -20 to 10°C. If the reaction temperature is too low, the solubility of the compound (3) may decrease, and if the reaction temperature is too high, the compound (3), the compound (4) or the metal complex (5) may be decomposed.

The reaction may be performed at a normal pressure or may be performed under a predetermined pressure. For example, the reaction may be performed by a method in which hydrogen gas is sealed into a rubber balloon and the balloon is connected to the opening of the container (a flask, etc.). Since hydrogen gas is consumed as the reaction proceeds, a sufficient amount of hydrogen gas is used, or hydrogen gas is supplemented during the reaction.

When the reaction is completed, a gas phase in the container is replaced with an inert gas, and as necessary, the metal complex is then removed by filtration, and can be purified by generally used purification methods such as extraction, crystallization, distillation, and various types of chromatography alone or an appropriate combination thereof.

### (Step 2)

The method according to the present embodiment may further comprise a step of reacting the compound (4) with a compound (6) to obtain a compound (7). In Formula (7), R¹ and Ar¹ have the same definitions as in Formula (4).

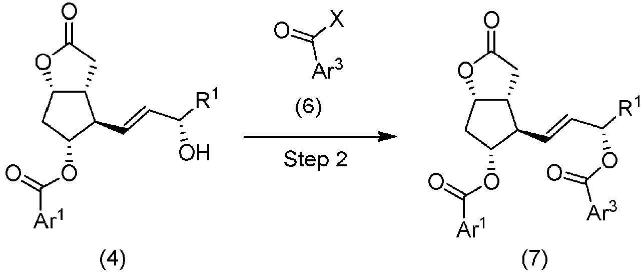

In Formula (6) or Formula (7), Ar³ is an aryl group that is optionally substituted. The aryl group that is optionally substituted in Ar³ may have the same definition as in Ar¹, but Ar¹ and Ar³ are preferably groups that are different from each other. A preferable Ar³ is an o-nitro phenyl group, a m-nitro phenyl group, or a p-nitro phenyl group.

The compound (6) is a reagent that acylates hydroxy groups of the compound (4). In Formula (6), X is a leaving group. Examples of leaving groups include a halogen atom (for example, a fluorine atom, a chlorine atom, and a bromine atom), and a relatively bulky acyloxy group (for example, a pivaloyloxy group).

In the step 2, for example, as necessary, a solvent, a base and an additive are added to the compound (4) in a predetermined container, the compound (6) is added thereto, and the mixture is then stirred for a predetermined time.

The reaction may be performed without a solvent or may be performed in a solvent. The reaction is preferably performed in an organic solvent. The solvent used may be any solvent in which the compound (4) and the compound (6) can be dissolved and which does not inhibit the reaction. Examples of solvents include aromatic hydrocarbons such as benzene, toluene, and xylene, aliphatic hydrocarbons such as hexane and heptane, carboxylate esters such as ethyl acetate and isopropyl acetate, halogenated hydrocarbons such as dichloromethane, 1,2-dichloroethane, chlorobenzene, and dichlorobenzene, and ethers such as diethyl ether, diisopropyl ether, tetrahydrofuran, methyl tert-butyl ether, cyclopentyl methyl ether, and 2-methyl-tetrahydrofuran. These solvents may be used alone or may be used by being selectively mixed. The amount of the solvent used is preferably an amount at which the molar concentration of the compound (4) is 0.2 to 1.2 mol/L.

In order to accelerate the reaction, a base may be added to the reaction solution. Examples of bases include trialkylamines such as triethylamine and N,N-diethyl-N-isopropylamine, and nitrogen-containing aromatic compounds such as pyridine and 2,6-dimethylpyridine. The amount of the base used varies depending on the amount of the compound (4), but is preferably 1 to 10 equivalents with respect to number of moles of the compound (4).

In addition, in order to further accelerate the reaction, an additive such as 4-dimethylaminopyridine, imidazole, and N-methylimidazole may be added thereto. The amount of the additive used varies depending on the amount of the compound (4) and the type of the base and the amount of the base used, but is preferably 0.001 to 0.05 equivalents with respect to the number of moles of the compound (4).

The reaction can be generally performed at a temperature applied for the acylation reaction. The reaction temperature may be, for example, -20 to 60°C, and is preferably 0 to 30°C.

The product can be purified by generally used purification methods such as extraction, crystallization, distillation, and various types of chromatography alone or an appropriate combination thereof.

### (Step 3)

The method according to the present embodiment may further comprise a step of converting the compound (7) into a compound (8) by treating it with a base in a solvent. In Formula (8), R¹ has the same definition as in Formula (7).

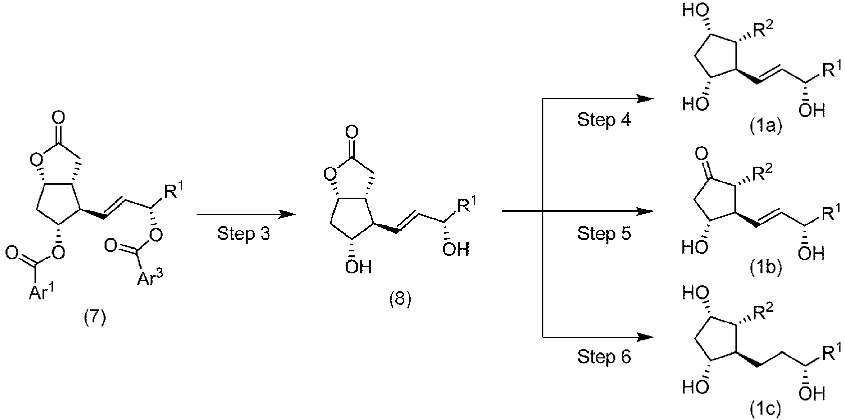

In the step 3, for example, a solvent is added to the compound (7), which are mixed in a predetermined container, a base is added thereto, and the mixture is then stirred for a predetermined time.

The base in the step 3 may be an organic base or an inorganic base. Examples of organic bases include trialkylamines such as triethylamine and N,N-diethyl-N-isopropylamine, nitrogen-containing aromatic compounds such as pyridine and 2,6-dimethylpyridine, and metal alkoxides such as sodium methoxide, potassium methoxide, sodium ethoxide, potassium ethoxide, sodium tert-butoxide, and potassium tert-butoxide. Examples of inorganic bases include potassium carbonate, sodium carbonate, cesium carbonate, potassium bicarbonate, sodium bicarbonate, potassium hydroxide, sodium hydroxide, tripotassium phosphate, trisodium phosphate, dipotassium hydrogen phosphate, disodium hydrogen phosphate, potassium dihydrogen phosphate, and sodium dihydrogen phosphate. The amount of the base used varies depending on the amount of the compound (7), but is preferably 1 to 10 equivalents with respect to the number of moles of the compound (7).

Examples of the solvent in the step 3 include aromatic hydrocarbons such as benzene, toluene, and xylene, aliphatic hydrocarbons such as hexane and heptane, halogenated hydrocarbons such as dichloromethane, 1,2-dichloroethane, chlorobenzene, and dichlorobenzene, ethers such as diethyl ether, diisopropyl ether, tetrahydrofuran, methyl tert-butyl ether, cyclopentyl methyl ether, and 2-methyl-tetrahydrofuran, alcohols such as methanol, ethanol, isopropyl alcohol, n-butyl alcohol, 2-butyl alcohol, tert-butyl alcohol, ethylene glycol, propylene glycol, 1,2-propanediol, and glycerin, acetonitrile, N,N-dimethylformamide (DMF), and N,N-dimethylacetamide. These solvents may be used alone or may be used by being selectively mixed. It preferably comprises water or an alcohol.

The amount of the solvent used is preferably an amount at which the molar concentration of the compound (7) is 0.05 to 1.0 mol/L and more preferably an amount at which the molar concentration of the compound (7) is 0.1 to 0.5 mol/L.

The reaction product can be purified by generally used purification methods such as extraction, crystallization, distillation, and various types of chromatography alone or an appropriate combination thereof.

The method according to the present embodiment may further comprise a step for converting the compound (8) into the compound (1a) or (1b). For such a step, the descriptions in Patent Literature 1 to 4 and Non-Patent Literature 1 can be referred to.

### (Step 4)

The step 4 is a step for obtaining the compound (1a), and may be composed of a plurality of steps. The compound (1a) may be, for example, prostaglandin F_{2α} derivatives such as dinoprost, and bimatoprost.

### (Step 5)

The step 5 is a step for obtaining a compound (1b), and may be composed of a plurality of steps. The compound (1b) may be, for example, prostaglandin E₁ derivatives such as alprostadil and limaprost, or prostaglandin E₂ derivatives such as dinoprostone.

### (Step 6)

The step 6 is a step for obtaining a compound (1c) and may be composed of a plurality of steps. The compound (1c) may be, for example, prostaglandin F_{2α} derivatives such as latanoprost.

Disclosed is a compound (7') which does not form part of the claimed invention.

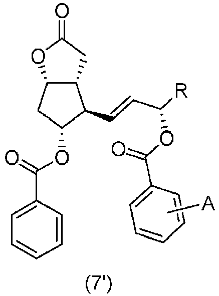

In Formula (7'), R is a C₁₋₈ alkyl group that is unsubstituted or substituted with a phenyl group. The definition of R is the same as the definition of R¹ in Formula (1a).

In Formula (7'), A is a nitro group bonded to any position of an ortho-position, a meta-position, and a para-position of a benzene ring.

The compound (7') may be a single diastereomer or may be a mixture of a plurality of diastereomers. In order to produce the compound (1a), (1b), or (1c), the compound (7') preferably has high optical purity.

The optical purity of the compound (7') can be improved by performing recrystallization. Examples of solvents used for crystallization include aromatic hydrocarbons such as benzene, toluene, and xylene, aliphatic hydrocarbons such as hexane and heptane, carboxylate esters such as ethyl acetate and isopropyl acetate, halogenated hydrocarbons such as dichloromethane, 1,2-dichloroethane, chlorobenzene, and dichlorobenzene, ethers such as diethyl ether, diisopropyl ether, tetrahydrofuran, methyl tert-butyl ether, cyclopentyl methyl ether, and 2-methyl-tetrahydrofuran, and alcohols such as methanol, ethanol, isopropyl alcohol, and 1-butanol. These solvents may be used alone or may be used by being selectively mixed. It preferably comprises carboxylate ester or an alcohol. A compound (7') having low optical purity is dissolved in a solvent while heating as necessary and gradually cooling, and thus a compound (7') having higher optical purity can be obtained.

The compound (7') has excellent crystallinity, and is useful as a production intermediate for producing the compound (1a), (1b), or (1c) having high optical purity.

### Examples

An embodiment of the present invention will be described in more detail with reference to examples.

The abbreviations used in the following description should be generally understood according to common general technical knowledge in the field. Specifically, the meanings of the abbreviations are as follows.
Bz: benzoyl
CAN: ammonium hexanitratocerate(IV)
CSA: 10-camphorsulfonic acid
DIBAL-H: diisobutylaluminum hydride
DMAP: 4-dimethylamino pyridine
DMF: N,N-dimethylformamide
Et: ethyl
IPA: isopropyl alcohol
n-: normal
NMR: nuclear magnetic resonance spectrum
p-: para
ph: phenyl
PMB: paramethoxybenzyl
Me-THF: 2-methyl-tetrahydrofuran
TEA: triethylamine
tert: tertiary
THF: tetrahydrofuran
THP: tetrahydropyran-2-yl

The ¹H-NMR spectrum is shown by a chemical shift value (δ) corrected with tetramethylsilane as an internal standard (0 ppm), and the split pattern is abbreviated as follows. s: singlet, d: doublet, t: triplet, q: quartet, dd: double doublet, ddd: double double doublet, dt: double triplet, m: multiplet, br: broad.

### Example 1: Method for producing dinoprostone

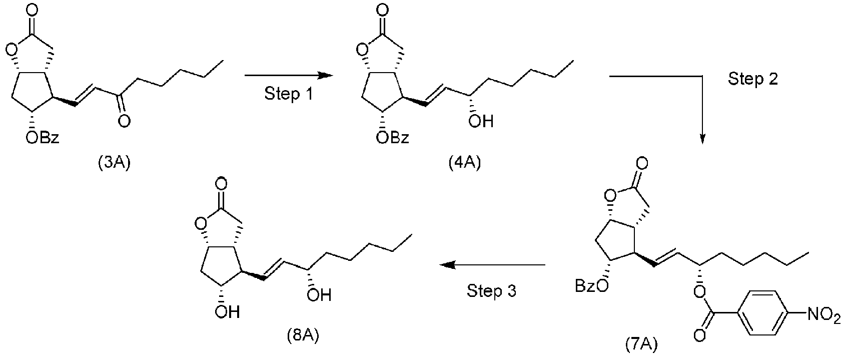

### (Step 1)

According to the methods described in Example 1 of Patent Literature 5 and Example 8 of Patent Literature 6, a compound (3A) was prepared from commercially available (-)-Corey Lactone-Benzoate (trade name) and dimethyl-2-oxo-heptylphosphonate. The compound (3A) (20.0 g, 54 mmol) and tripotassium phosphate (1.1 g) were suspended in a mixed solution containing THF (100 mL) and ethanol (20 mL). A gas phase in a container under an ice bath was replaced with nitrogen gas. (R)-RUCY-xylBINAP (commercially available from Tokyo Chemical Industry Co., Ltd., 0.64 g, 0.52 mmol) was added to the suspension, a gas phase was then replaced with hydrogen gas, and the mixture was stirred for 24 hours. After a gas phase was replaced with nitrogen gas, the solvent was distilled off under a reduced pressure, and thereby a compound (4A) (a diastereomer excess rate of 87%) was obtained. The obtained compound (4A) was used in the step 2 without further purification.

### (Step 2)

Ethyl acetate (50 mL) was added to the compound (4A) to dissolve it, and TEA (10.9 g) and DMAP (33 mg) were added thereto. An ethyl acetate solution (40 mL) containing p-nitrobenzoyl chloride (14.0 g) was added dropwise to the obtained solution, and the mixture was stirred for 1 hour. Ethyl acetate was added to the reaction solution, washing with water was performed, and an organic layer was collected. After the solvent was distilled off under a reduced pressure, ethanol (100 mL) was added to the obtained residue, and dissolved while heating, and the mixture was gradually cooled to 0°C to obtain crystals. The obtained crystals were washed with ethanol and dried under a reduced pressure, and thereby a compound (7A) (21.1 g, 40 mmol, 74% yield, a diastereomer excess rate of 99%) was obtained.
mp: 105°C;
¹HNMR (400 MHz, CDCl₃): δ 8.26-8.21(m, 2H), 8.14-8.09(m, 2H), 7.91(dd, 2H), 7.53(dd, 1H), 7.38(dd, 2H), 5.76-5.65(m, 2H), 5.46(ddd, 1H), 5.25(ddd, 1H), 5.06(ddd, 1H), 2.90-2.74(m, 3H), 2.61(ddd, 1H), 2.51(d, 1H), 2.22(ddd, 1H), 1.74(m, 2H), 1.36-1.26(m, 6H), 0.86(t, 3H); ¹³CNMR (100 MHz, CDCl₃): δ 176.1, 165.8, 163.8, 150.4, 135.7, 133.3, 132.0, 131.3, 130.6, 129.5, 129.4, 128.4, 123.5, 83.0, 78.5, 75.8, 53.9, 42.4, 37.5, 34.7, 34.2, 31.4, 24.8, 22.4, 13.9

### (Step 3)

A methanol solution (100 mL) containing the compound (7A) (20.0 g, 38 mmol) was ice-cooled, potassium carbonate (10.6 g) was added thereto, and the mixture was reacted for 4 hours. Phosphoric acid diluted with water was added, and washing with a toluene/hexane mixed solution (volume ratio of 1/1) was performed. The solvent was distilled off under a reduced pressure, the obtained residue was extracted with ethyl acetate, and an organic layer was collected. The solvent of the organic layer was distilled off under a reduced pressure to obtain a compound (8A) (9.8 g, 37 mmol, 95% yield).
¹HNMR (400 MHz, CDCl₃): δ 5.60(dd, 1H), 5.44(dd, 1H), 4.88(ddd, 1H), 4.08-4.03(m, 1H), 3.97-3.91(m, 1H), 2.78-2.69(m, 1H), 2.62-2.49(m, 2H)2.47-2.38(m, 2H), 2.31-2.22(m, 2H), 1.98-1.91(m, 1H), 1.63-1.42(m, 2H), 1.38-1.23(m, 6H), 0.89(t, 3H);
¹³CNMR (100 MHz, CDCl₃): δ 176.9, 136.8, 130.2, 82.4, 76.3, 72.8, 56.12, 42.34, 39.61, 37.05, 34.0, 31.6, 25.1, 22.5, 13.9)

### (Step 5)

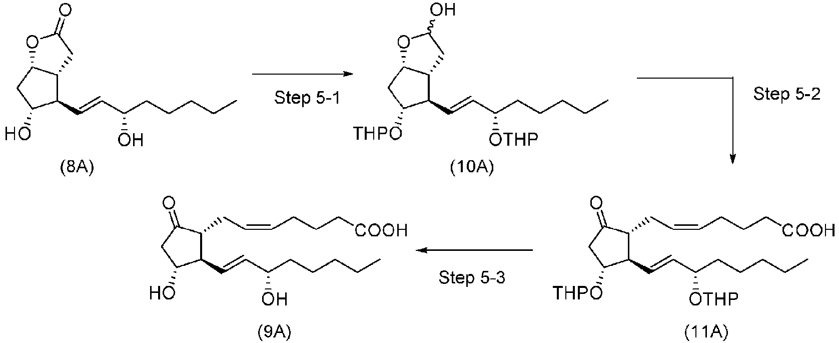

According to the method described in Example 14 of Patent Literature 7, the compound (8A) (9.8 g, 37 mmol) was converted into a compound (10A). The compound (10A) was converted into a compound (11A) according to the method described in Example 1 of Patent Literature 8, and also converted into a compound (9A) (7.2 g, 19 mmol, 51% yield (Step 3)) with reference to the method described in Example 14 of Patent Literature 9.
mp: 66°C
¹HNMR (400 MHz, CDCl₃): δ 5.68(dd, 1H), 5.58(dd, 1H), 5.47-5.34(m, 2H), 4.20-4.10(m, 1H), 4.05(dd, 1H), 2.75(dd, 1H), 2.47-1.93(m, 9H), 1.82-1.43(m, 4H), 1.40-1.20(m, 6H), 0.89(t, 3H)
¹³CNMR (100 MHz, CDCl₃): δ 214.4, 177.6, 136.7, 130.9, 126.9, 73.2, 72.5, 54.6, 53.6, 46.4, 37.2, 33.2, 31.8, 26.4, 25.3, 24.6, 22.8, 14.2

In the step 1, catalytic hydrogen reduction was performed in the same manner under the following conditions. The reaction conditions and the results are shown in Table 1.

**[Table 1]**

| | Metal complex | | Reducing agent | Base | Solvent | Reaction temperature (°C) | %d.e. |
|---|---|---|---|---|---|---|---|
| | Type | Amount used | | | | | |
| Example 2 | (R)-RUCY-xylBINAP | 3 mol% | hydrogen gas | K₃PO₄ | THF/water (5:1) | 0 | 90 |
| Example 3 | (R)-RUCY-xylBINAP | 3 mol% | hydrogen gas | K₃PO₄ | THF/IPA (1:1) | 0 | 88 |
| Example 4 | (R)-RUCY-xylBINAP | 1 mol% | hydrogen gas | K₃PO₄ | THF/ ethanol (5:1) | -10 | 94 |
| Example 5 | (R)-RUCY-xylBINAP | 3 mol% | hydrogen gas | K₃PO₄ | CPME/IPA (1:10) | 25 | 85 |
| Example 6 | (R)-RUCY-xylBINAP | 3 mol% | hydrogen gas | K₃PO₄ | IPA | 25 | 85 |
| Example 7 | (R)-RUCY-xylBINAP | 3 mol% | hydrogen gas | Cs₂CO₃ | THF/IPA (1:10) | 25 | 87 |
| Example 8 | (R)-RUCY-xylBINAP | 3 mol% | hydrogen gas | K₃PO₄ | Me-THF/IPA (2:20) | 25 | 86 |
| Comparative Example 1 | RuCl[(R,R)-Tsdpen](p-cymene) | 1 mol% | formic acid | TEA | THF | 60 | 16 |
| Comparative Example 2 | RuCl[(R,R)-Tsdpen](mesitylene) | 1 mol% | formic acid | TEA | THF | 60 | 9 |
| Comparative Example 3 | Ru[(R,R)-Tsdpen](p-cymene) | 1 mol% | formic acid | TEA | THF | 60 | 20 |
| Comparative Example 4 | RuCl[(R,R)-Msdpen](p-cymene) | 1 mol% | formic acid | TEA | THF | 60 | 36 |
| Comparative Example 5 | R(R,R)-Ts-DENEB | 1 mol% | formic acid | TEA | THF | 25 | -13 |

### Example 9: Method for producing limaprost

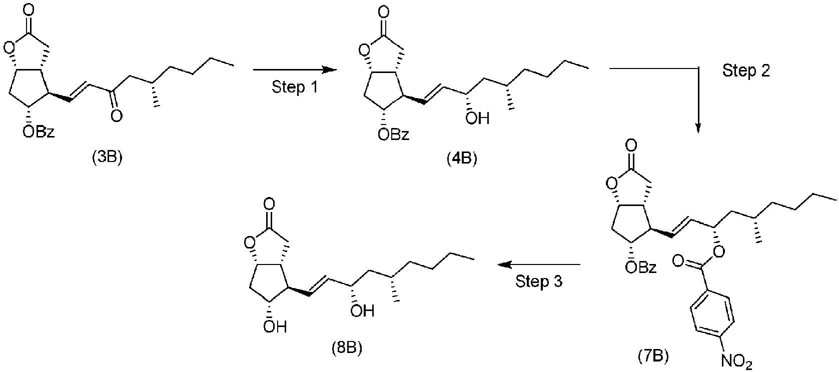

### (Step 1)

With reference to the method described in Patent Literature 5 and 6, a compound (3B) was prepared from commercially available (-)-Corey Lactone-Benzoate (trade name) and dimethyl-2-oxo-4-methyloctylphosphonate.
¹HNMR (300 MHz, CDCl₃): δ 8.02-7.96(m, 2H), 7.62-7.55(m, 1H), 7.50-7.42(m, 2H), 6.68(dd, 1H), 6.24(d, 1H), 5.33(q, 1H), 5.14-5.08(m, 1H), 2.96-2.85(m, 3H), 2.68-2.28(m, 5H), 2.05-1.93(m, 1H), 1.32-1.15(m, 6H), 0.92-0.84(m, 6H)

With reference to the method described in Example 1, a compound (4B) (a diastereomer excess rate of 87%) was obtained from the compound (3B) (20.0 g, 50 mmol).

### (Step 2)

With reference to the method described in Example 1, a compound (7B) (18.1g, 33 mmol, 66% yield, a diastereomer excess rate of 97%) was obtained from the compound (4B).
mp: 74°C;
¹HNMR (400 MHz, CDCl₃): δ 8.22-8.19(m, 2H), 8.10-8.07(m, 2H), 7.90-7.88(m, 2H), 7.52-7.49(m, 1H), 7.37-7.33(m, 2H), 5.77-5.71(m, 1H), 5.67-5.61(m, 1H), 5.53(dd, 1H), 5.23(dd, 1H), 5.04(ddd, 1H), 2.89-2.71(m, 3H), 2.59(ddd, 1H), 2.49(d, 1H), 2.20(ddd, 1H), 1.64(t, 2H), 1.49-1.41(m, 1H), 1.26-1.10(m, 6H), 0.91(d, 3H), 0.85(t, 3H);
¹³CNMR (100 MHz, CDCl₃): δ 176.2, 165.9, 163.9, 150.5, 135.9, 133.4, 132.4, 131.5, 130.7, 129.6, 128.5, 123.6, 83.1, 78.6, 74.7, 54.0, 42.5, 41.5, 37.6, 36.6, 34.8, 29.5, 29.0, 23.0, 19.9, 14.2

### (Step 3)

With reference to the method described in Example 1, a compound (8B) (9.2 g, 31 mmol, 95% yield) was obtained from the compound (7B) (18.1 g, 33 mmol).
¹HNMR (400 MHz, CDCl₃): δ 5.56(dd, 1H), 5.44(dd, 1H), 4.88(ddd, 1H), 4.17-4.08(m, 1H), 3.96-3.89(m, 1H), 2.78-2.21(m, 6H), 1.93(ddd, 1H), 1.87(br, 1H), 1.63(Br, 1H), 1.45-1.08(m, 9H), 0.93-0.82(m, 6H)

### (Step 5)

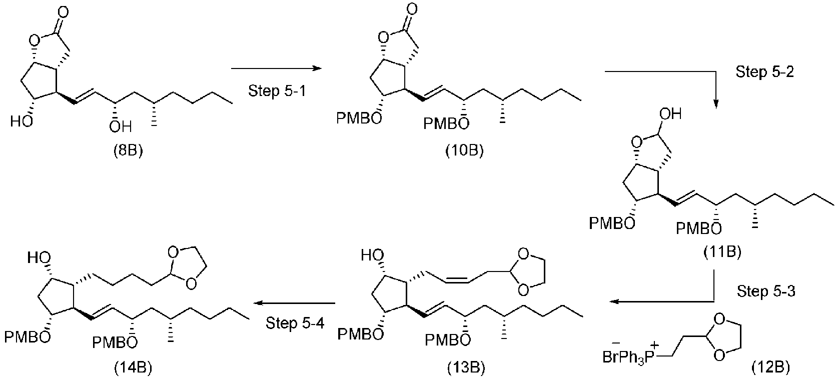

### (Step 5-1)

### (3aR,4R,5R,6aS)-5-[(4-methoxybenzyl)oxy]-4-{(3S,5S,E)-3-[(4-metho xybenzyl)oxy]-5-methylnon-1-en-1-yl}hexahydro-2H-cyclopenta[b]fura n-2-one (10B)

Dichloromethane (3 mL) was added to the compound (8B) (0.29 g, 0.98 mmol) to dissolve it, 4-methoxybenzyl 2, 2, 2-trichloroacetoimidate (0.69 g) and CSA (0.01 g) were added thereto, and the mixture was stirred at room temperature. A saturated sodium bicarbonate aqueous solution and ethyl acetate were added to the reaction solution, the mixture was filtered with diatomite, and the solvent was distilled off under a reduced pressure. The residue was purified through supercritical fluid chromatography (device name: Viridis CSH Fluoro-Phenyl OBD Prep Column, carbon dioxide/methanol/chloroform=90/5/5 to 80/10/10), and thereby a compound (10B) (0.374 g, 71% yield) was obtained.
¹HNMR (400 MHz, CDCl₃): δ 7.25-7.16(m, 4H), 6.88-6.83(m, 4H), 5.48(dd, 1H), 5.41(dd, 1H), 4.98-4.93(m, 1H), 4.57-4.21(m, 4H), 3.81-3.73(m, 8H), 2.79-2.61(m, 3H), 2.53-2.46(m, 1H), 2.34-2.11(m, 2H), 1.55-1.02(m, 7H), 0.91-0.82(m, 6H)

### (Step 5-2)

### (3aR,4R,5R,6aS)-5-[(4-methoxybenzyl)oxy]-4-{(3S,5S,E)-3-[(4-metho xybenzyl)oxy]-5-methylnon-1-en-1-yl}hexahydro-2H-cyclopenta[b]fura n-2-ol (11B)

The compound (10B) (0.370 g, 0.69 mmol) was dissolved in dichloromethane (7 mL), and DIBAL-H (1 mol/L hexane solution, 0.758 mL) was gently added dropwise thereto at -78°C. The mixture was stirred at -78°C for 30 minutes, a saturated Rochelle salt aqueous solution was then added, the mixture was stirred at room temperature for 30 minutes, ethyl acetate was added thereto, the mixture was filtered with diatomite, and the solvent was distilled off under a reduced pressure. The residue was purified through silica gel column chromatography (heptane/ethyl acetate=2/1 to 1/1), and thereby a compound (11B) (0.348 g, 94% yield) was obtained.
¹HNMR (400 MHz, CDCl₃): δ 7.25-7.18(m, 4H), 6.87-6.79(m, 4H), 5.68-5.36(m, 3H), 4.68-4.20(m, 5H), 3.81-3.69(m, 8H), 2.52-2.17(m, 4H), 2.06-1.77(m, 2H), 1.54-1.36(m, 3H), 1.29-0.99(m, 7H), 0.91-0.82(m, 6H)

### (Step 5-3)

### (1S,2R,3R,4R)-2-[(Z)-4-(1,3-dioxolane-2-yl)but-2-en-1-yl]-4-[(4-metho xybenzyl)oxy]-3-{(3S,5S,E)-3-[(4-methoxybenzyl)oxy]-5-methylnon-1-en-1-yl}cyclopentanol (13B)

The compound (12B) (2-(1,3-dioxolane-2-yl)ethyltriphenylphosphonium bromide, 1.40 g, 3.16 mmol) was suspended in THF (6 mL), potassium tert-butoxide (0.354 g, 3.16 mmol) was added, and the mixture was stirred at room temperature for 30 minutes. A THF (2 mL) solution containing the compound (11B) (0.340 g, 0.631 mmol) was added dropwise to a brown suspension reaction solution under ice-cooling and the mixture was stirred at 0°C for 30 minutes. A saturated ammonium chloride aqueous solution and ethyl acetate were added to the reaction solution, the mixture was filtered with diatomite, and the solvent was distilled off under a reduced pressure. The residue was purified through silica gel column chromatography (heptane/ethyl acetate=4/1 to 2/1, and then chloroform/methanol=50/1 to 30/1), and thereby a compound (13B) (0.258 g, 66% yield) was obtained as a white solid.
¹HNMR (400 MHz, CDCl₃): δ 7.25-7.18(m, 4H), 6.86-6.79(m, 4H), 5.56-5.40(m, 4H), 4.93-4.89(m, 1H), 4.54-4.22(m, 4H), 4.13-4.07(m, 1H), 4.01-3.83(m, 4H), 3.81-3.74(m, 8H), 3.01-2.97(m, 1H), 2.68-2.55(m, 2H), 2.45-2.34(m, 2H), 2.18-1.99(m, 2H), 1.90-1.83(m, 1H), 1.56-1.37(m, 4H), 1.30-1.01(m, 6H), 0.89-0.82(m, 6H)

### (Step 5-4)

### (1S,2R,3R,4R)-2-[4-(1,3-dioxolane-2-yl)butyl]-4-[(4-methoxybenzyl)ox y]-3-{(3S,5S,E)-3-[(4-methoxybenzyl)oxy]-5-methylnon-1-en-1-yl}cycl opentanol (14B)

The compound (13B) (0.250 g, 0.401 mmol) was dissolved in ethyl acetate (3 mL), RhCl(PPh₃)₃ (0.074 g, 0.080 mmol) was added, and the mixture was stirred under a hydrogen atmosphere at room temperature for 5 hours. The reaction solution was concentrated under a reduced pressure, and the obtained residue was purified through silica gel column chromatography (heptane/ethyl acetate=4/1 to 2/1), and then amino silica gel column chromatography (heptane/ethyl acetate=4/1 to 2/1), and thereby a compound (14B) (0.208 g, 83% yield) was obtained as a white solid.
¹HNMR (400 MHz, CDCl₃): δ 7.25-7.18(m, 4H), 6.87-6.81(m, 4H), 5.48(dd, 1H), 5.38(dd, 1H), 4.86-4.81(m, 1H), 4.52-4.21(m, 4H), 4.18-4.12(m, 1H), 3.98-3.91(m, 2H), 3.86-3.73(m, 10H), 2.54-2.47(m, 1H), 2.20-1.95(m, 3H), 1.72-1.02(m, 18H), 0.91-0.81(m, 6H)

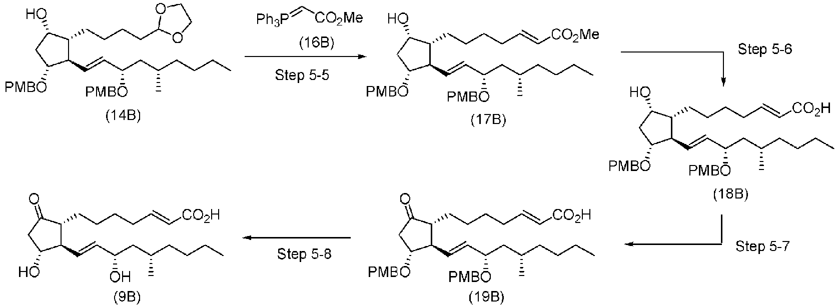

### (Step 5-5)

### (E)-methyl 7-((1R,2R,3R,5S)-5-hydroxy-3-[(4-methoxybenzyl)oxy]-2-{(3S,5S,E)-3 -[(4-methoxybenzyl)oxy]-5-methylnon-1-en-1-yl}cyclopentyl)hept-2-en oate (17B)

The compound (14B) (0.190 g, 0.304 mmol) was dissolved in THF (4 mL), 6 mol/L hydrochloric acid (2 mL) was added under ice-cooling, and the mixture was stirred at room temperature overnight. A saturated sodium bicarbonate aqueous solution was added to the reaction solution and extraction with ethyl acetate was performed. An organic layer was washed with a saturated saline solution and dried with anhydrous magnesium sulfate, and the solvent was distilled off under a reduced pressure. The residue was purified through silica gel column chromatography (heptane/ethyl acetate=2/1 to 1/1), and thereby a crude purified product of a compound (15B) was obtained as a white solid. The obtained crude purified product was dissolved in dichloromethane (4 mL), methyl(triphenylphosphoraniliden)acetate (16B) (0.508 g, 1.52 mmol) was added under ice-cooling, and the mixture was stirred at room temperature for 2 hours. The reaction solution was purified through silica gel column chromatography (heptane/ethyl acetate=2/1 to 1/1), and thereby a compound (17B) (0.131 g, 68% yield) was obtained as a white solid.

### (Step 5-6)

### (E)-7-((1R,2R,3R,5S)-5-hydroxy-3-[(4-methoxybenzyl)oxy]-2-{(3S,5S, E)-3-[(4-methoxybenzyl)oxy]-5-methylnon-1-en-1-yl}cyclopentyl)hept-2-enoic acid (18B)

The compound (17B) (0.0900 g, 0.141 mmol) was suspended in ethanol (2 mL), a 1 mol/L potassium hydroxide aqueous solution (0.565 mL, 0.565 mmol) was added under ice-cooling, and the mixture was stirred at 40°C or 3 hours. 1mol/L hydrochloric acid (0.60 mL) and ethyl acetate were added to the reaction solution under ice-cooling, and the mixture was filtered with diatomite. The solvent was distilled off under a reduced pressure. The residue was purified through silica gel column chromatography (heptane/ethyl acetate=4/1 to 2/1, and then chloroform/methanol=20/1 to 10/1), and thereby a compound (18B) (0.0826 g, 94% yield) was obtained as a white solid.
¹HNMR (400 MHz, CDCl₃): δ 7.24-7.17(m, 4H), 7.04(dt, 1H), 6.87-6.81(m, 4H), 5.81(dt, 1H), 5.49(dd, 1H), 5.39(dd, 1H), 4.53-4.23(m, 4H), 4.17-4.12(m, 1H), 3.86-3.72(m, 8H), 2.55-2.46(m, 1H), 2.26-2.19(m, 2H), 2.02-1.93(m, 2H), 1.68-1.03(m, 17H), 0.91-0.81(m, 6H)

### (Step 5-7)

### (E)-7-((1R,2R,3R)-3-[(4-methoxybenzyl)oxy]-2-{(3S,5S,E)-3-[(4-meth oxybenzyl)oxy]-5-methylnon-1-en-1-yl}-5-oxocyclopentyl)hept-2-enoic acid (19B)

The compound (18B) (80.0 mg, 0.128 mmol) was dissolved in dichloromethane (1 mL), Dess-Martin Periodinane (109 mg, 0.257 mmol) was added under ice-cooling, and the mixture was stirred at room temperature for 2 hours. Water and ethyl acetate were added to the reaction solution, the mixture was filtered with diatomite, and the solvent was distilled off under a reduced pressure. The residue was purified through silica gel column chromatography (chloroform/methanol=20/1 to 10/1, and then heptane/ethyl acetate=1/1), and thereby a compound (19B) (80.0 mg, quantitative yield) was obtained as colorless and transparent oil.
¹HNMR (400 MHz, CDCl₃): δ 7.24-7.18(m, 4H), 7.02(dt, 1H), 6.87-6.80(m, 4H), 5.80(dt, 1H), 5.61(dd, 1H), 5.52(dd, 1H), 4.56-4.25(m, 4H), 3.92-3.75(m, 8H), 2.77-2.59(m, 2H), 2.28-2.16(m, 3H), 2.02-1.94(m, 1H), 1.69-1.03(m, 18H), 0.90-0.83(m, 6H)

### (Step 5-8)

### (E)-7-{(1R,2R,3R)-3-hydroxy-2-[(3S,5S,E)-3-hydroxy-5-methylnon-1-e n-1-yl]-5-oxocyclopentyl}hept-2-enoic acid (9B)

The compound (19B) (10.0 mg, 16.0 µmol) was dissolved in a mixed solvent containing acetonitrile (0.5 mL) and water (0.05 mL), CAN (35.3 mg, 64.0 µmol) was added under ice-cooling, and the mixture was stirred at room temperature for 30 minutes. Under ice-cooling, ethyl acetate was added, and extraction in a saturated sodium bicarbonate aqueous solution was performed. 1mol/L hydrochloric acid was added to an aqueous layer until the liquid reached a pH of 5, and extraction with ethyl acetate was performed. An organic layer was washed with a saturated saline solution and dried with anhydrous magnesium sulfate, and the solvent was distilled off under a reduced pressure. The residue was purified through silica gel column chromatography (chloroform/methanol=20/1 to 10/1), and thereby a compound (9B) (4.4 mg, 74% yield) was obtained.
¹HNMR (400 MHz, CDCl₃): δ 7.00(dt, 1H), 5.81(d, 1H), 5.66(dd, 1H), 5.56(dd, 1H), 4.25-4.17(m, 1H), 4.11-4.01(m, 1H), 3.40(br, 1H), 2.75(dd, 1H), 2.41-2.32(m, 1H), 2.27-2.14(m, 3H), 2.08-1.97(m, 1H), 1.67-1.11(m, 16H), 0.95-0.84(m, 6H)

### Example 10: Method for producing latanoprost

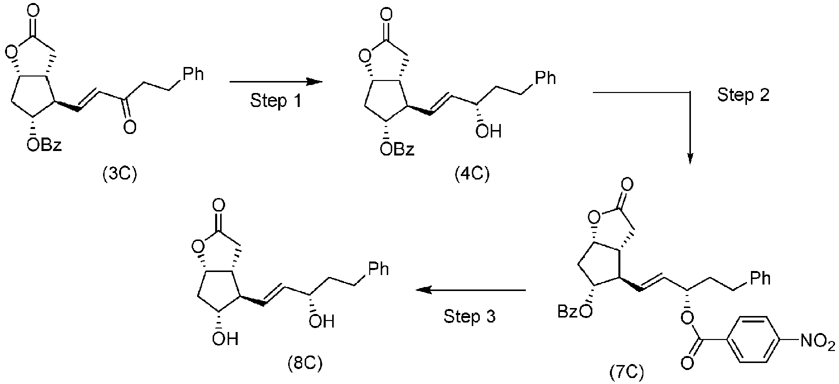

### (Step 1)

With reference to the method described in Patent Literature 5, a compound (3C) was prepared from commercially available (-)-Corey Lactone-Benzoate (trade name) and dimethyl-2-oxo-4-phenylbutylphosphonate. In the same manner as in Example 1, a compound (4C) (a diastereomer excess rate of 80%) was obtained from the compound (3C) (20.0 g, 49 mmol).

### (Step 2)

In the same manner as in Example 1, a compound (7C) (20.0 g, 36 mmol, 74% yield (Step 2), a diastereomer excess rate of 99%) was obtained from the compound (4C).
¹HNMR (400 MHz, CDCl₃): δ 8.22-8.19(m, 2H), 8.07-8.03(m, 2H), 7.91-7.89(m, 2H), 7.52-7.48(m, 1H), 7.37-7.33(m, 2H), 7.26-7.17(m, 2H), 7.16-7.10(m, 3H), 5.74-5.67(m, 2H), 5.51-5.46(m, 1H), 5.25-5.21(m, 1H), 5.05-5.01(m, 1H)2.88-2.46(m, 7H), 2.23-1.99(m, 3H);
¹³CNMR (100 MHz, CDCl₃): δ 176.2, 165.9, 163.8, 150.6, 140.8, 135.6, 133.4, 132.5, 130.9, 130.7, 129.6, 129.4, 128.7, 128.5, 128.4, 126.3, 123.6, 83.1, 78.6, 77.5, 77.1, 76.8, 75.3, 54.0, 42.5, 37.5, 35.8, 34.8, 31.6

### (Step 3)

A compound (8C) was obtained from the compound (7C) according to the method described in Patent Literature 1.

### (Step 6)

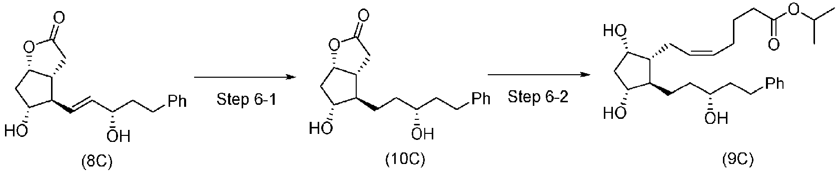

According to the method described in Example 7 of Patent Literature 5, a compound (10C) was obtained from the compound (8C) (5.0 g, 9.0 mmol). In addition, according to the method described in Patent Literature 4, a compound (9C) (1.1 g, 2.5 mmol, 28% yield (Step 3)) was obtained from the compound (10C).
¹HNMR (400 MHz, CDCl₃): δ 7.29-7.23(m, 2H), 7.21-7.17(m, 3H), 5.48-5.43(m, 1H), 5.41-5.36(m, 1H), 4.99(m, 1H), 4.16(m, 1H), 3.94(m, 1H), 3.66(m, 1H), 2.82-2.77(m, 3H), 2.70-2.09(m, 8H)1.90-1.31(m, 12H), 1.22(d, 6H);
¹³CNMR (100 MHz, CDCl₃): δ 173.5, 142.1, 129.6, 129.3, 128.4, 125.8, 78.8, 74.7, 71.3, 67.7, 52.9, 51.9, 42.5, 39.0, 35.8, 34.0, 32.1, 29.6, 26.9, 26.6, 24.9, 21.8.

## Claims

1. A method for producing a compound represented by Formula (1a), (1b), or (1c), comprising: 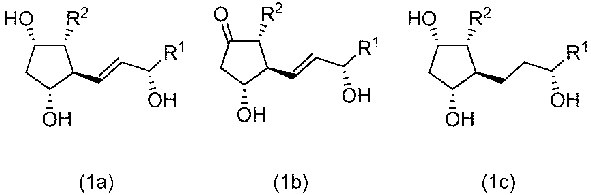
in the formula, R¹ is a C₁₋₈ alkyl group that is optionally substituted with a phenyl group, R² is a group represented by Formula (2a), (2b) or (2c): 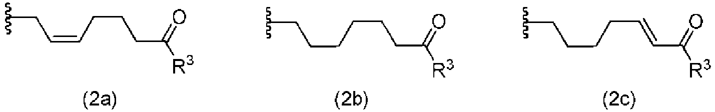
in the formula, R³ is a hydroxy group, a C₁₋₃ alkoxy group, a mono(C₁₋₃ alkyl)amino group or a di(C₁₋₃ alkyl)amino group;
the method comprising a step of reducing a compound represented by Formula (3) in the presence of a metal complex represented by Formula (5), an inorganic base, and a solvent under a hydrogen atmosphere to obtain a compound represented by Formula (4): 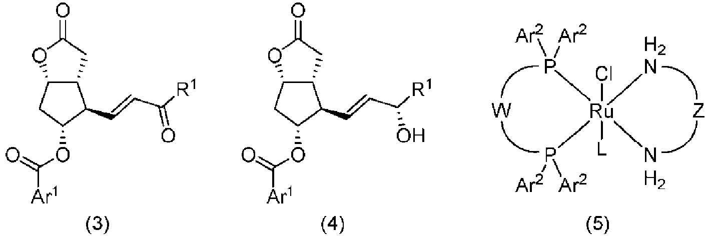
in the formula, Ar¹ is an aryl group that is optionally substituted,
each Ar² is independently a phenyl group, a 3,5-dimethylphenyl group, or a 3,5-di(tert-butyl)-4-methoxyphenyl group,
W is a biphenyl group that is optionally substituted or a binaphthyl group that is optionally substituted,
Z is an ethylene group that is substituted with two or more groups selected from among a phenyl group, a C₁₋₃ alkoxyphenyl group and a C₁₋₈ alkyl group, and L is a chlorine atom, or if Z has a phenyl group or a C₁₋₃ alkoxyphenyl group, L is one of carbon atoms constituting the phenyl group or the C₁₋₃ alkoxyphenyl group.

2. The method according to claim 1, wherein the solvent comprises at least one solvent selected from among ethers, alcohols, and acetonitrile.

3. The method according to claim 1 or 2, wherein Ar¹ is a phenyl group, and Ar² is a 3,5-dimethylphenyl group.

4. The method according to any one of claims 1 to 3, wherein R¹ is a n-pentyl group, a 2-methylhexyl group, or a 2-phenylethyl group.

5. The method according to any one of claims 1 to 4, further comprising
a step of reacting a compound represented by Formula (4) with a compound represented by Formula (6) to obtain a compound represented by Formula (7): 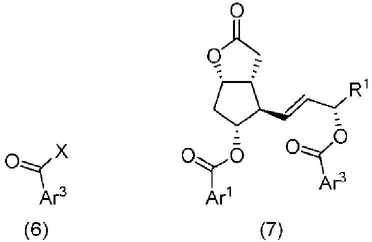 in the formula, R¹ is a C₁₋₈ alkyl group that is optionally substituted with a phenyl group, Ar¹ and Ar³ are each independently an aryl group, and X is a leaving group.

## Patentansprüche

1. Ein Verfahren zur Herstellung einer Verbindung, dargestellt durch Formel (1a), (1b) oder (1c), umfassend: 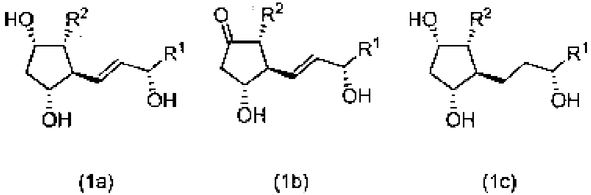
in der Formel ist R¹ eine C₁₋₈-Alkylgruppe, die gegebenenfalls mit einer Phenylgruppe substituiert ist, R² ist eine Gruppe, dargestellt durch Formel (2a), (2b) oder (2c): 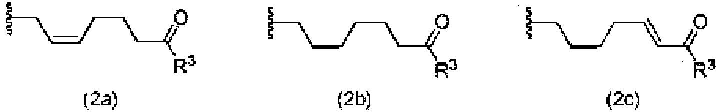
in der Formel ist R³ eine Hydroxygruppe, eine C₁₋₃-Alkoxygruppe, eine Mono(C₁₋₃-alkyl)aminogruppe oder eine Di(C₁₋₃-alkyl)aminogruppe;
wobei das Verfahren einen Schritt des Reduzierens einer Verbindung, dargestellt durch Formel (3), in Gegenwart eines Metallkomplexes, dargestellt durch Formel (5), einer anorganischen Base und eines Lösungsmittels unter einer Wasserstoffatmosphäre umfasst, um eine Verbindung, dargestellt durch Formel (4), zu erhalten: 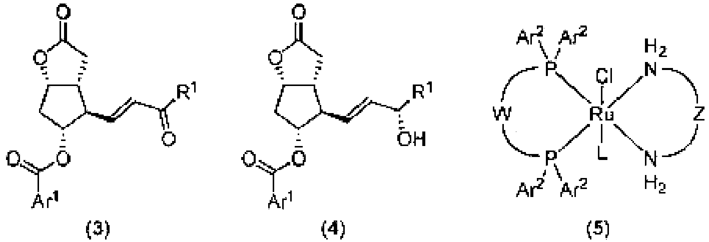
in der Formel ist Ar¹ eine Arylgruppe, die gegebenenfalls substituiert ist,
jedes Ar² ist unabhängig eine Phenylgruppe, eine 3,5-Dimethylphenylgruppe oder eine 3,5-Di(tert-butyl)-4-methoxyphenylgruppe,
W ist eine Biphenylgruppe, die gegebenenfalls substituiert ist, oder eine Binaphthylgruppe, die gegebenenfalls substituiert ist,
Z ist eine Ethylengruppe, die mit zwei oder mehr Gruppen, ausgewählt aus einer Phenylgruppe, einer C₁₋₃-Alkoxyphenylgruppe und einer C₁₋₈-Alkylgruppe, substituiert ist, und L ist ein Chloratom oder, wenn Z eine Phenylgruppe oder eine C₁₋₃-Alkoxyphenylgruppe aufweist, ist L eines der Kohlenstoffatome, die die Phenylgruppe oder die C₁₋₃-Alkoxyphenylgruppe bilden.

2. Das Verfahren nach Anspruch 1, wobei das Lösungsmittel mindestens ein Lösungsmittel, ausgewählt aus Ethern, Alkoholen und Acetonitril, umfasst.

3. Das Verfahren nach Anspruch 1 oder 2, wobei Ar¹ eine Phenylgruppe ist und Ar² eine 3,5-Dimethylphenylgruppe ist.

4. Das Verfahren nach einem der Ansprüche 1 bis 3, wobei R¹ eine n-Pentylgruppe, eine 2-Methylhexylgruppe oder eine 2-Phenylethylgruppe ist.

5. Das Verfahren nach einem der Ansprüche 1 bis 4, ferner umfassend
einen Schritt des Umsetzens einer Verbindung, dargestellt durch Formel (4), mit einer Verbindung, dargestellt durch Formel (6), um eine Verbindung, dargestellt durch Formel (7), zu erhalten: 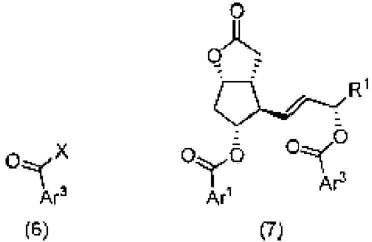 in der Formel ist R¹ eine C₁₋₈-Alkylgruppe, die gegebenenfalls mit einer Phenylgruppe substituiert ist, Ar¹ und Ar³ sind jeweils unabhängig eine Arylgruppe und X ist eine Abgangsgruppe.

## Revendications

1. Méthode de production d'un composé représenté par la Formule (1a), (1b), ou (1c), comprenant : 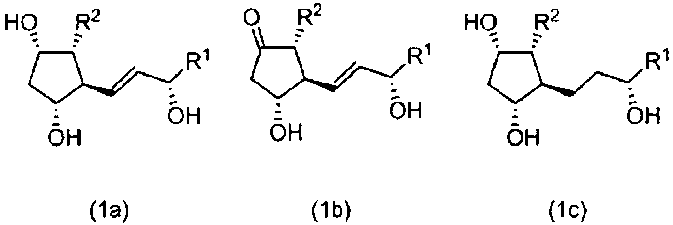
dans la formule, R¹ est un groupe alkyle en C₁₋₈ qui est éventuellement substitué par un groupe phényle, R² est un groupe représenté par la Formule (2a), (2b) ou (2c) : 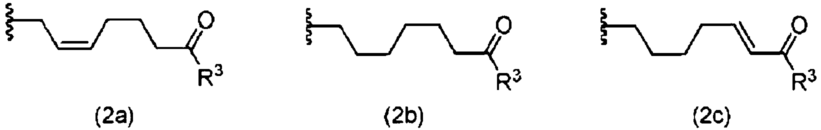 dans la formule, R³ est un groupe hydroxy, un groupe alcoxy en C₁₋₃, un groupe mono(alkyle en C₁₋₃)amino ou un groupe di(alkyle en C₁₋₃)amino ;
la méthode comprenant une étape de réduction d'un composé représenté par la Formule (3) en la présence d'un complexe métallique représenté par la Formule (5), d'une base inorganique, et d'un solvant sous une atmosphère d'hydrogène pour obtenir un composé représenté par la Formule (4) : 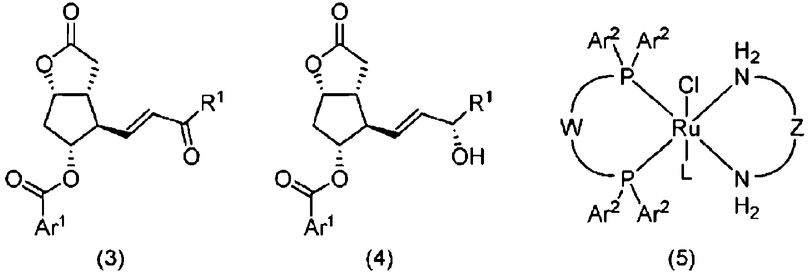 dans la formule, Ar¹ est un groupe aryle qui est éventuellement substitué,
chaque Ar² est indépendamment un groupe phényle, un groupe 3,5-diméthylphényle, ou un groupe 3,5-di(tert-butyl)-4-méthoxyphényle,
W est un groupe biphényle qui est éventuellement substitué ou un groupe binaphtyle qui est éventuellement substitué,
Z est un groupe éthylène qui est substitué par deux groupes ou plus, choisis parmi un groupe phényle, un groupe (alcoxy en C₁₋₃)phényle et un groupe alkyle en C₁₋₈, et L est un atome de chlore, ou si Z a un groupe phényle ou un groupe (alcoxy en C₁₋₃)phényle, L est l'un des atomes de carbone constituant le groupe phényle ou le groupe (alcoxy en C₁₋₃)phényle.

2. Méthode selon la revendication 1, dans laquelle le solvant comprend au moins un solvant choisi parmi les éthers, les alcools et l'acétonitrile.

3. Méthode selon la revendication 1 ou 2, dans laquelle Ar¹ est un groupe phényle, et Ar² est un groupe 3,5-diméthylphényle.

4. Méthode selon l'une quelconque des revendications 1 à 3, dans laquelle R¹ est un groupe n-pentyle, un groupe 2-méthylhexyle, ou un groupe 2-phényléthyle.

5. Méthode selon l'une quelconque des revendications 1 à 4, comprenant en outre
une étape réactionnelle d'un composé représenté par la Formule (4) avec un composé représenté par la Formule (6) pour obtenir un composé représenté par la Formule (7) : 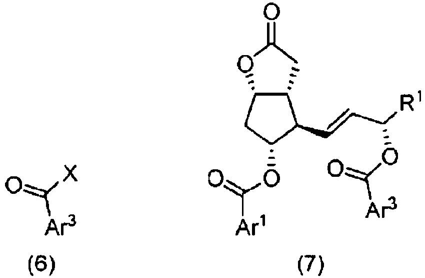 dans la formule, R¹ est un groupe alkyle en C₁₋₈ qui est éventuellement substitué par un groupe phényle, Ar¹ et Ar³ sont chacun indépendamment un groupe aryle, et X est un groupe partant.
